# EUROPÄISCHE PATENTSCHRIFT

(11) **EP 3 310 242 B1**
(45) Veröffentlichungstag und Bekanntmachung des Hinweises auf die Patenterteilung: **05.08.2020**
(21) Anmeldenummer: 16727195.6
(22) Anmeldetag: 06.06.2016
(51) Int. Cl.: A61B 1/00

(54) **CHIRURGISCHES INSTRUMENT, INSBESONDERE URETEROSKOP**
SURGICAL INSTRUMENT, IN PARTICULAR URETEROSCOPE
INSTRUMENT CHIRURGICAL, EN PARTICULIER URÉTÉROSCOPE

(30) Priorität: 22.06.2015 DE 102015211424
(43) Veröffentlichungstag der Anmeldung: 25.04.2018
(73) Patentinhaber: Olympus Winter & Ibe GmbH, 22045 Hamburg (DE)
(72) Erfinder: KIEDROWSKI, Gregor, 22397 Hamburg (DE)
(74) Vertreter: Seemann & Partner Patentanwälte mbB
(86) Internationale Anmeldenummer: PCT/EP2016/062744
(87) Internationale Veröffentlichungsnummer: WO 2016/206956

(56) Entgegenhaltungen:
- EP-A1- 2 229 871
- WO-A1-2014/111946
- DE-A1- 3 916 288
- DE-A1-102010 054 422
- US-A- 4 630 598
- US-A1- 2008 208 001

## Beschreibung

Die Erfindung betrifft ein chirurgisches Instrument, insbesondere Ureteroskop, mit einem langgestreckten, insbesondere starren, Schaftrohr zur Aufnahme von wenigstens zwei Arbeitskanälen und mit wenigstens zwei im Schaftrohr angeordneten Arbeitskanälen, wobei wenigstens im distalen Endbereich des Schaftrohrs die Arbeitskanäle im Querschnitt jeweils eine Profilkante oder eine Profilnase aufweisen.

In der Urologie werden Ureteroskope zu endoskopischen Arbeiten im Ureter und im Nierenbecken eingesetzt. Um endoskopisch in diesen Bereich zu gelangen, wird das Ureteroskop von außen durch die Urethra (Harnröhre) in die Blase vorgeschoben, von dort durch das Ostium (Mündung des Ureters mit Verschlussklappe) in den Ureter (Harnleiter) eingeführt und in diesem bis zum Operationsgebiet vorgeschoben, beispielsweise bis zu einem Stein oder einer Verengung im Ureter bzw. bis zum Nierenbecken.

Das Schaftrohr des Ureteroskopes hat typischerweise eine Länge von mindestens ungefähr 400 mm. Da der Ureter und insbesondere das Ostium sehr eng sind, sollte der Schaftdurchmesser nicht grösser sein als etwa 3 bis 4 mm. Daraus ergibt sich eine extrem lange und dünne Konfiguration des Schaftrohrs.

Das Schaftrohr eines Ureteroskopes sollte steif sein, um das Dirigieren des distalen Endes vom proximalen Ende her zu ermöglichen, insbesondere bei der schwierigen Einführung in das Ostium. Das Schaftrohr ist daher in der Regel starr und weist ein äußeres Metallrohr auf, das ihm die notwendige Steifigkeit vermittelt und das die innenliegenden Teile des Schaftrohrs flüssigkeitsdicht umschließt.

Im Inneren des Schaftrohrs sind wenigstens eine Betrachtungseinrichtung vorgesehen sowie Lichtleiter zur Beleuchtung und vorzugsweise ein oder zwei Arbeitskanäle, der bzw. die das Einführen von Instrumenten, wie Katheter, Zangen, Steinfangkörbe, Lithotriptoren (Steinzertrümmerungsgeräte) etc. ermöglichen.

In WO 2014/111946 A1 ist eine Abdichtungsvorrichtung für ein Koloskopieverfahren offenbart, wobei die Abdichtungsvorrichtung ein Dichtungsrohr aufweist. Im Dichtungsrohr ist hierbei ein langgestreckter Träger angeordnet. In einer Ausgestaltung verfügt der Träger über mehrere Kanäle, wobei ein Arbeitskanal einen kreisförmigen Querschnitt aufweist.

Außerdem ist in DE 10 2010 054 422 A1 ein Endoskop beschrieben, das einen Arbeitskanal aufweist. In dem Arbeitskanal ist ein Einsatz einsetzbar, so dass zwei Arbeitskanäle in einem Lumen und einem Lumen ausgebildet sind. Durch den Einsatz werden somit zwei Arbeitskanäle bereitgestellt.

Überdies ist in DE 39 16 288 A1 ein Uretero-Renoskop beschrieben, das einen schlauchförmigen Katheterkörper mit einer Anzahl von Kanälen aufweist. Hierbei sind ein Katheterkopfstück und der Katheterkörper wenigstens vierkanalig ausgebildet, wobei zur Ausbildung der Kanäle im Inneren des Katheterkörpers zwei sich im Wesentlichen senkrecht kreuzende Trennwandungen vorgesehen sind.

Ferner offenbart EP 2 229 871 A1 ein medizinisches Instrument, insbesondere Hysteroskop, wobei der Schaft des Instruments einen dreieckförmigen Querschnitt aufweist.

Des Weiteren offenbart US 4,630,598 A ein Uretero-Renoskop mit einem ovalen Schaft, der durch einen Innenschaft unterteilt ist. Ferner sind im Innenschaft eine Optik und ein Hilfsinstrument angeordnet, wobei die verbleibenden Querschnitte in Unterteilungen Kanäle für den Zulauf und den Ablauf von Spülflüssigkeit bilden.

Außerdem zeigt US 2008/0208001 A1 ein flexibles bzw. nachgiebiges Endoskop, wobei der Endoskopschaft im Wesentlichen D-förmig in einer Ausgestaltung ausgebildet ist.

Beispielsweise ist ein Ureteroskop in DE 10 2004 059 255 B3 beschrieben.

Darüber hinaus sind auch Ureteroskope mit zwei Arbeitskanälen bekannt, so dass durch die beiden Arbeitskanäle sich Instrumente einführen lassen, wodurch die Vielseitigkeit und Flexibilität beim Einsatz der Ureteroskope erhöht ist.

Ausgehend vom diesem Stand der Technik besteht die Aufgabe der Erfindung darin, die Handhabung bei Ureteroskopen mit zwei Arbeitskanälen auf einfache Weise zu verbessern.

Die Aufgabe wird gelöst durch ein chirurgisches Instrument, insbesondere Ureteroskop, mit einem langgestreckten, insbesondere starren, Schaftrohr zur Aufnahme von wenigstens zwei Arbeitskanälen und mit wenigstens zwei im Schaftrohr angeordneten Arbeitskanälen, wobei wenigstens im distalen Endbereich des Schaftrohrs die Arbeitskanäle im Querschnitt jeweils eine Profilkante oder eine Profilnase aufweisen, das dadurch weitergebildet ist, dass wenigstens im distalen Endbereich das Schaftrohr im Querschnitt eine Polygonform aufweist oder nach Art einer Polygonform ausgebildet ist, wobei im Inneren das Schaftrohr eine Schaftrohrkontaktfläche im Querschnitt mit einem ebenen Schaftrohrkontaktflächenabschnitt und einem gekrümmten Schaftrohrkontaktflächenabschnitt für die wenigstens zwei Kanäle aufweist, wobei die Arbeitskanäle jeweils eine der Schaftrohrkontaktfläche zugewandte Arbeitskanalanlagefläche aufweisen, wobei die Arbeitskanalanlagefläche der Arbeitskanäle mit jeweils einem Schaftrohrkontaktflächenabschnitt für eine Arbeitskanalanlagefläche verlötet sind, wobei die Arbeitskanäle derart ausgebildet sind, dass jeder Arbeitskanal mit einem ebenen Schaftrohrkontaktflächenabschnitt und einem gekrümmten Schaftrohrkontaktflächenabschnitt verbunden ist.

Die Erfindung basiert auf dem Gedanken, dass durch die Ausbildung der im Schaftrohr des chirurgischen Instruments angeordneten zwei Arbeitskanäle mit einer Profilnase oder Profilkante im Querschnitt, vorzugsweise am distalen Endes des Schaftrohrs, die Arbeitskanäle eine unrunde, d.h. nicht-kreisförmige oder nicht-ovale, Geometrie aufweisen, so dass sie an die Innenkontur des Schaftrohrs in einem Bereich angepasst sind, wobei vorzugsweise der Querschnitt der distalen Innenkontur des Schaftrohrs nach Art eines Polygons ausgebildet ist. Insbesondere ist gemäß einem weiteren Aspekt der Erfindung vorgesehen, dass die Innenkontur und die Außenkontur des Schaftrohres im Querschnitt eine Polygonform oder ein Polygonprofil aufweisen, wobei die Polygonform oder das Polygonprofil vorzugsweise gerundete Ecken aufweist.

Im Gegensatz zum Stand der Technik, bei dem die für Instrumente vorgesehenen Arbeitskanäle (kreis-)runde Querschnitte aufweisen, ist es erfindungsgemäß möglich, dass die erfindungsgemäßen Arbeitskanäle mit jeweils einer Profilkante oder einer Profilnase ausgebildet sind, so dass die Arbeitskanäle platzsparend bei gleichzeitiger Reduzierung der u.U. zu verfüllenden Zwischenräume zwischen den Arbeitskanälen sowie zwischen den Arbeitskanälen und dem Schaftrohr angeordnet werden.

Dazu ist weiter vorgesehen, dass die Arbeitskanäle jeweils eine dem anderen Arbeitskanal zugewandte, vorzugsweise ebene und/oder formkomplementäre, Kontaktfläche aufweisen, wodurch der Zwischenraum sowie der Abstand zwischen den beiden Arbeitskanälen verkleinert wird, da insbesondere die einander zugewandten Kontaktflächen der Arbeitskanäle formkomplementär zusammenwirken. Insbesondere sind die einander zugewandten Kontaktflächen eben ausgebildet.

Vorzugsweise sind außerdem die einander zugewandten Kontaktflächen der Arbeitskanäle miteinander verbunden, insbesondere verlötet. Durch das Verbinden bzw. das Verlöten der Arbeitskanäle wird der Zwischenraum zwischen den Arbeitskanälen fluiddicht verfüllt. Insbesondere sind für das Verlöten der Arbeitskanäle die Arbeitskanäle aus Metall hergestellt.

Eine weitere Ausführungsform des chirurgischen Instruments zeichnet sich dadurch aus, dass die Arbeitskanäle jeweils ein Lumen aufweisen, wobei insbesondere die Lumen der Arbeitskanäle unterschiedlich sind.

Das Schaftrohr des chirurgischen Instruments, insbesondere des Ureteroskops, ist vorzugsweise starr ausgebildet, wobei es in einer weiteren Ausgestaltung vorgesehen ist, dass das Schaftrohr, in dem die beiden Arbeitskanäle angeordnet sind, über die gesamte Länge das Schaftrohr im Querschnitt eine Polygonform aufweist oder nach Art einer Polygonform ausgebildet ist.

Vorzugsweise weist das Schaftrohr in einer Ausgestaltung im Querschnitt eine dreieckförmige Polygonform auf. Hierbei ist es im Rahmen der Erfindung möglich, dass die dreieckförmige Polygonform gerundete Ecken aufweist. Ferner ist es in einer Ausgestaltung möglich, dass die Polygonform im Querschnitt asymmetrisch ist.

Die Arbeitskanäle, die jeweils eine Profilkante oder eine Profilnase aufweisen, sind hierbei im Schaftrohr an der Schaftrohrkontaktfläche angeordnet, wodurch Zwischenräume sowie der Abstand zwischen den Arbeitskanälen und der Innenseite des Schaftrohrs verkleinert sind.

Um eine raumsparende Ausbildung des chirurgischen Instruments zu erreichen, ist vorgesehen, dass die Arbeitskanäle jeweils eine der Schaftrohrkontaktfläche zugewandte Arbeitskanalanlagefläche aufweisen, wobei die äußeren Arbeitskanalanlageflächen der Arbeitskanäle mit jeweils einem Schaftrohrkontaktflächenabschnitt für eine Arbeitskanalanlagefläche verlötet sind. Dadurch, dass einerseits die Arbeitskanäle miteinander über ihre einander zugewandten Kontaktflächen verbunden, insbesondere verlötet, sind, und andererseits die Arbeitskanäle jeweils eine der Innenseite des Schaftrohrs zugewandte Arbeitskanalanlagefläche aufweisen und die Arbeitskanalanlageflächen der Arbeitskanäle mit der Schaftrohrkontaktfläche verbunden sind, sind keine Hohlräume in der Anordnung der Arbeitskanäle im Schaftrohr zwischen den Arbeitskanälen und dem Schaftrohr vorhanden. Insbesondere sind die Arbeitskanäle derart ausgebildet, dass jeder Arbeitskanal mit einen ebenen Schaftrohrkontaktflächenabschnitt und einem gekrümmten Schaftrohrkontaktflächenabschnitt verbunden ist, wodurch ein bzw. jeder Arbeitskanal einer rundgeformten Ecke des im Querschnitt polygonartigen Schaftrohrs zugeordnet ist.

Vorteilhafterweise sind gemäß einer Ausgestaltung die Arbeitskanäle und das Schaftrohr jeweils als Metallrohre ausgebildet. Außerdem ist vorzugsweise das chirurgische Instrument als Ureteroskop ausgebildet.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch eine Seitenansicht eines Ureteroskopes;
- Fig. 2: schematisch ein Querschnitt durch das Schaftrohr entlang der Linie A-A in Fig. 1.

In den Zeichnungen sind jeweils gleiche oder gleichartige Elemente und/oder Teile mit denselben Bezugsziffern versehen, so dass von einer erneuten Vorstellung jeweils abgesehen wird.

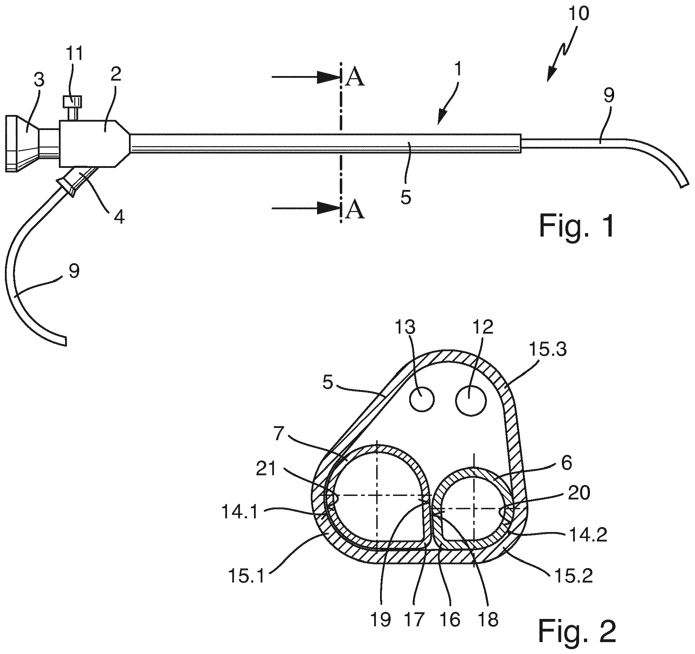

In Fig. 1 ist schematisch ein starres Ureteroskop 10 dargestellt. Es weist ein Schaftteil 1 auf, an dessen proximalem Ende ein Hauptkörper 2 befestigt ist mit Okular 3 und Einführungsteil 4. Der Schaftteil 1 weist ein den Außenumfang bestimmendes äußeres Schaftrohr 5 auf.

Im Inneren des Schaftteiles 1 sind zwei innere Arbeitskanäle 6 und 7 angeordnet (vgl. Fig. 2), durch die hindurch Instrumente eingeführt werden. Im Raum zwischen den Arbeitskanälen 6 und 7 und dem Schaftrohr 5 sind ferner ein Lichtleiter 13 (vgl. Fig.2) und ein Optikkanal mit einer Optik 12 (vgl. Fig. 2) angeordnet, so dass ein Bild von einem am distalen Ende des Schaftteiles 1 angeordneten Objektiv zum Okular 3 übertragen wird.

Die Arbeitskanäle 6 und 7 umschließen jeweils ein Lumen, durch das ein Instrument 9 durch das Einführungsteil 4 hindurch in einen der Arbeitskanäle 6, 7 geschoben werden kann. Im dargestellten Beispiel ist das Instrument 9 ein Laserlithotriptor, dessen proximal vorgesehener Lasergenerator nicht dargestellt ist. In den anderen, freien Arbeitskanal kann ferner ein weiteres Instrument eingeführt werden.

Der Raum zwischen den inneren Arbeitskanälen 6 und 7 und dem Schaftrohr 5 ist unter anderem mit einem (hier nicht dargestellten) Lichtleiter ausgefüllt, der z.B. aus Lichtleiterfasern gebildet ist und zur Beleuchtung des Betrachtungsfeldes dient. Am proximalen Ende ist der Lichtleiter über einen Lichtleiteranschluss 11 aus dem Hauptkörper 2 herausgeführt. Dort kann ein Lichtleiterverbindungskabel zu einer nicht dargestellten Lichtquelle angeschlossen werden.

Die Länge des Schaftteiles 1 beträgt bei einem Ureteroskop beispielsweise 400 bis 500 mm. Der Außendurchmesser des Schaftteiles 1, also der Außendurchmesser des äußeren Schaftrohrs 5 beträgt maximal etwa 4 mm.

Wie aus der schematischen Darstellung in Fig. 2 ersichtlich ist, hat das Schaftrohr 5 im Querschnitt eine dreieckförmige Polygonform, wobei die dreieckförmige, asymmetrische Polygonform gerundete Ecken 15.1, 15.2, 15.3 aufweist. Im Schaftrohr 5 sind eine Optik 12 und ein Lichtleiter 13 angeordnet, der sich in Längsrichtung des Schaftrohrs 5 vom proximalen zum distalen Ende erstreckt.

Die Arbeitskanäle 6, 7 sind im Querschnitt jeweils mit einer Profilkante 16 bzw. 17 ausgebildet, wodurch die Querschnitte der Arbeitskanäle 6 und 7 eine Art Tropfenform aufweisen. Die Profilkanten 16, 17 der Arbeitskanäle 6, 7 sind einander zugewandt und einander gegenüberliegend angeordnet. Ferner weisen dadurch die Querschnitte der Arbeitskanäle 6 und 7 einander zugewandte ebene Kontaktflächen 18 und 19 auf, die miteinander durch Lot oder dergleichen verbunden sind. Im Rahmen der Erfindung ist in einer Ausgestaltung vorgesehen, dass die Querschnitte der Arbeitskanäle 6, 7 nur im distalen Bereich die Profilkanten 16, 17 aufweisen. Die Profilkanten 16, 17 der Arbeitskanäle 6, 7 können beispielsweise durch ein Aufdornen der Querschnittsform ausgebildet werden.

Die tropfenförmige Form der Querschnitte der Arbeitskanäle 6, 7 ist an die jeweilige Kontur der Ecken 15.1, 15.2, in denen die Arbeitskanäle angeordnet sind, angepasst, sodass die Arbeitskanäle 6, 7 mit ihren äußeren Anlageflächen 20, 21 für die Ecken 15.1, 15.2 mit jeweils einer den Anlageflächen 20,21 zugewandten Schaftrohrkontaktfläche 14.1, 14.2 verbunden bzw. verlötet sind. Hierbei ist es vorgesehen, dass die Schaftrohrkontaktflächen 14.1, 14.2 sowohl ebene Schaftrohrkontaktflächenabschnitte und gekrümmte Schaftrohrkontaktflächenabschnitte aufweisen, die mit den Arbeitskanälen 6,7 verbunden sind.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 1: Schaftteil
- 2: Hauptkörper
- 3: Okular
- 4: Einführungsteil
- 5: Schaftrohr
- 6: Arbeitskanal
- 7: Arbeitskanal
- 9: Instrument
- 10: Ureteroskop
- 11: Lichtleiteranschluss
- 12: Optik
- 13: Lichtleiter
- 14.1, 14.2: Schaftrohrkontaktfläche
- 15.1, 15.2, 15.3: Ecke
- 16: Profilkante
- 17: Profilkante
- 18: Kontaktfläche
- 19: Kontaktfläche
- 20: Anlagefläche
- 21: Anlagefläche

## Patentansprüche

1. Chirurgisches Instrument (10), insbesondere Ureteroskop, mit einem langgestreckten, insbesondere starren, Schaftrohr (5) zur Aufnahme von wenigstens zwei Arbeitskanälen (6, 7) und mit wenigstens zwei im Schaftrohr (5) angeordneten Arbeitskanälen (6, 7), wobei wenigstens im distalen Endbereich des Schaftrohrs (5) die Arbeitskanäle (6, 7) im Querschnitt jeweils eine Profilkante (16, 17) oder eine Profilnase aufweisen, **dadurch gekennzeichnet, dass** wenigstens im distalen Endbereich das Schaftrohr (5) im Querschnitt eine Polygonform aufweist oder nach Art einer Polygonform ausgebildet ist, wobei im Inneren das Schaftrohr (5) eine Schaftrohrkontaktfläche (14.1, 14.2) im Querschnitt mit einem ebenen Schaftrohrkontaktflächenabschnitt und einem gekrümmten Schaftrohrkontaktflächenabschnitt für die wenigstens zwei Arbeitskanäle (6, 7) aufweist, wobei die Arbeitskanäle (6, 7) jeweils eine der Schaftrohrkontaktfläche (14.1, 14.2) zugewandte Arbeitskanalanlagefläche (20, 21) aufweisen, wobei die Arbeitskanalanlageflächen (20, 21) der Arbeitskanäle (6, 7) mit jeweils einem Schaftrohrkontaktflächenabschnitt für eine Arbeitskanalanlagefläche (20, 21) verlötet sind, wobei die Arbeitskanäle (6, 7) derart ausgebildet sind, dass jeder Arbeitskanal (6, 7) mit einem ebenen Schaftrohrkontaktflächenabschnitt und einem gekrümmten Schaftrohrkontaktflächenabschnitt verbunden ist.

2. Chirurgisches Instrument (10) nach Anspruch 1, **dadurch gekennzeichnet, dass** die Arbeitskanäle (6, 7) jeweils eine dem anderen Arbeitskanal (6, 7) zugewandte, vorzugsweise ebene und/oder formkomplementäre, Kontaktfläche (18, 19) aufweisen.

3. Chirurgisches Instrument (10) nach Anspruch 2, **dadurch gekennzeichnet, dass** die einander zugewandten Kontaktflächen (18, 19) der Arbeitskanäle (6, 7) miteinander verbunden, insbesondere verlötet, sind.

4. Chirurgisches Instrument (10) nach einem der Ansprüche 1 bis 3, **dadurch gekennzeichnet, dass** die Arbeitskanäle (6, 7) jeweils ein Lumen aufweisen, wobei insbesondere die Lumen der Arbeitskanäle (6, 7) unterschiedlich sind.

5. Chirurgisches Instrument (10) nach einem der Ansprüche 1 bis 4, **dadurch gekennzeichnet, dass** das Schaftrohr (5) im Querschnitt eine dreieckförmige Polygonform aufweist.

6. Chirurgisches Instrument (10) nach einem der Ansprüche 1 bis 5, **dadurch gekennzeichnet, dass** die Arbeitskanäle (6, 7) und das Schaftrohr (5) jeweils als Metallrohre ausgebildet sind.

## Claims

1. A surgical instrument (10), in particular a ureteroscope, with an elongated, in particular rigid, shaft tube (5) for receiving at least two working channels (6, 7) and with at least two working channels (6, 7) arranged in the shaft tube (5), wherein at least in the distal end area of the shaft tube (5), the working channels (6, 7) in cross-section each comprise a profile edge (16, 17) or a profile nose, **characterized in that** at least in the distal end area, the shaft tube (5) in cross-section comprises a polygonal shape or is formed in a polygonal shape manner, wherein inside the shaft tube (5) comprises a shaft tube contact surface (14.1, 14.2) in cross-section with a planar shaft tube contact surface section and a curved shaft tube contact surface section for the at least two working channels (6, 7), wherein the working channels (6, 7) each comprise a working channel bearing surface (20, 21) facing the shaft tube contact surface (14.1, 14.2), wherein the working channel bearing surfaces (20, 21) of the working channels (6, 7) are soldered with a shaft tube contact surface section for a working channel bearing surface (20, 21), wherein the working channels (6, 7) are formed such that each working channel (6, 7) is connected to a planar shaft tube contact surface section and a curved shaft tube contact surface section.

2. The surgical instrument (10) according to claim 1, **characterized in that** the working channels (6, 7) each comprise a contact surface (18, 19) facing the other, preferably planar and/or complementary in shape, working channel (6, 7).

3. The surgical instrument (10) according to claim 2, **characterized in that** the interfacing contact surfaces (18, 19) of the working channels (6, 7) are interconnected, in particular soldered together.

4. The surgical instrument (10) according to one of claims 1 to 3, **characterized in that** the working channels (6, 7) each comprise a lumen, wherein in particular the lumina of the working channels (6, 7) are different.

5. The surgical instrument (10) according to one of claims 1 to 4, **characterized in that** the shaft tube (5) comprises a triangular polygonal shape in cross-section.

6. The surgical instrument (10) according to one of claims 1 to 5, **characterized in that** the working channels (6, 7) and the shaft tube (5) are respectively formed as metal tubes.

## Revendications

1. Instrument chirurgical (10), en particulier urétéroscope, avec un tube (5) formant tige allongé, en particulier rigide, destiné à recevoir au moins deux canaux de travail (6, 7) et avec au moins deux canaux de travail (6, 7) disposés dans le tube (5) formant tige, les canaux de travail (6, 7), disposés au moins dans la zone d'extrémité distale du tube (5) formant tige, présentant chacun un bord profilé (16, 17) ou un nez profilé en section transversale, **caractérisé en ce qu'**au moins dans la zone d'extrémité distale, le tube (5) formant tige présente une forme polygonale en section transversale ou est réalisé à la manière d'un polygone, le tube (5) formant tige présentant à l'intérieur de lui une surface de contact (14.1, 14.2) en section transversale avec une partie plate de surface de contact de tube et une partie courbée de surface de contact de tube pour lesdits au moins deux canaux de travail (6, 7), les canaux de travail (6, 7) présentant chacun une surface (20, 21) de contact de canal de travail tournée vers la surface (14.1, 14.2) de contact du tube, les surfaces (20, 21) de contact des canaux de travail que comprennent les canaux de travail (6, 7) étant chacune soudées à une partie de surface de contact de tube pour une surface (20, 21) de contact de canal de travail, les canaux de travail (6, 7) étant conçus de telle manière que chaque canal de travail (6, 7) est relié à une partie plate de surface de contact de tube et à une partie courbe de surface de contact de tube.

2. Instrument chirurgical (10) selon la revendication 1, **caractérisé en ce que** les canaux de travail (6, 7) présentent chacun une surface de contact (18, 19) de préférence plane et/ou de forme complémentaire, tournée vers l'autre canal de travail (6, 7).

3. Instrument chirurgical (10) selon la revendication 2, **caractérisé en ce que** les surfaces de contact (18, 19) des canaux de travail (6, 7) qui se font face sont reliées l'une à l'autre, en particulier sont soudées.

4. Instrument chirurgical (10) selon l'une quelconque des revendications 1 à 3, **caractérisé en ce que** les canaux de travail (6, 7) présentent chacun une lumière, les lumières des canaux de travail (6, 7) étant notamment différentes.

5. Instrument chirurgical (10) selon l'une des revendications 1 à 4, **caractérisé en ce que** le tube (5) formant tige présente une forme polygonale triangulaire en section transversale.

6. Instrument chirurgical (10) selon l'une des revendications 1 à 5, **caractérisé en ce que** les canaux de travail (6, 7) et le tube (5) formant tige sont chacun sous la forme de tubes métalliques.
